# EUROPÄISCHE PATENTSCHRIFT

(11) **EP 1 354 205 B1**
(45) Veröffentlichungstag und Bekanntmachung des Hinweises auf die Patenterteilung: **16.01.2008**
(21) Anmeldenummer: 01967000.9
(22) Anmeldetag: 11.08.2001
(51) Int. Cl.: G01N 33/68

(54) **VERFAHREN ZUR PROGNOSE DES KUMULATIVEN ÜBERLEBENS VON PATIENTEN**
METHODS FOR THE PROGNOSIS OF THE CUMULTATIVE SURVIVAL OF PATIENTS
PROCEDE POUR LE PROGNOSTIC DE LA SURVIE CUMULTATIVE DES CLIENTELES

(30) Priorität: 18.08.2000 DE 10040904
(43) Veröffentlichungstag der Anmeldung: 22.10.2003
(73) Patentinhaber: Germed IQ GmbH, 20148 Hamburg (DE)
(72) Erfinder: Böger, Rainer H., Prof. Dr., 20148 Hamburg (DE)
(74) Vertreter: Kloiber, Thomas
(86) Internationale Anmeldenummer: PCT/DE2001/003089
(87) Internationale Veröffentlichungsnummer: WO 2002/014873

(56) Entgegenhaltungen:
- WO-A-00/46395
- WO-A-98/49199

## Beschreibung

Die Erfindung betrifft Verfahren zum Nachweis einer Wahrscheinlichkeit des zukünftigen Auftretens oder Fortschreitens von Erkrankungen, die mit einer Störung des NO-Stoffwechsels einhergehen. Darüberhinaus betrifft die Erfindung Mittel zum Nachweis von endogenen Methyl-Argininen in biologischen Flüssigkeiten.

Die endogenen Methyl-Arginine ADMA und SDMA sind Derivate der Aminosäure L-Arginin. L-Arginin ist die Vorstufe zur Bildung von Stickstoffmonoxid (NO) im menschlichen Körper. NO wiederum ist ein wichtiger physiologischer Mediator im Herz-Kreislaufsystem und in anderen Organsystemen, der an der Regulation von Blutdruck und Gefäßwiderstand, Adhäsion und Aggregation von Thrombozyten, Adhäsion von Leukozyten und Monozyten und der Proliferation glatter Gefäßmuskelzellen beteiligt ist (Böger et al., Atherosclerosis 1996; 127: 1-11). NO spielt auch eine wichtige physiologische Rolle bei der Erektion. Bei Herz-Kreislauferkrankungen wie Arteriosklerose, Hypercholesterinämie, Hypertonie, chronischer Herzinsuffizienz, bei Stoffwechselerkrankungen wie Diabetes mellitus, bei Präeklampsie, bei erektiler Dysfunktion und anderen Erkrankungen kommt es zur Abschwächung der biologischen Wirkungen von NO, wodurch das Fortschreiten dieser Erkrankungen und der begleitenden Gefäßläsionen beschleunigt wird. Durch Gabe von L-Arginin kann diesem Geschehen entgegengewirkt werden.

In mehreren klinischen und experimentellen Untersuchungen konnte gezeigt werden, daß es bei den genannten Erkrankungen zu einem Ansteigen der Konzentration des endogenen L-Arginin-Analogons ADMA im Plasma oder Serum kommen kann. Erhöhte Konzentrationen von ADMA wurden gefunden bei peripherer arterieller Verschlußerkrankung (Böger et al., Circulation 1997; 95: 2068-2074), bei Hypercholesterinämie (Böger et al., Circulation 1998; 98: 1842-1847), bei Hypertonie (Surdacki et al., J. Cardiovasc. Pharmacol. 1999; 33: 652-658), bei chronischer Niereninsuffizienz (Kielstein et al., J. Am. Soc. Nephrol. 1999; 10: 594-600) und bei chronischer Herzinsuffizienz. Eine Ursachen-Wirkungsbeziehung zwischen erhöhten ADMA-Konzentrationen und diesen Erkrankungen konnte aus den Ergebnissen dieser Studien jedoch nicht abgeleitet werden. ADMA ist ein Inhibitor der Bildung von NO aus L-Arginin, die durch das Enzym NO-Synthase in Endothelzellen vermittelt wird. Somit wäre erklärlich, daß die erhöhten Konzentrationen von ADMA durch Hemmung der NO-Bildung zum Fortschreiten des Krankheitsprozesses beitragen könnten. Auch erhöhte Blut-Glucose-Konzentrationen, wie sie beim Diabetes mellitus auftreten, führen zu verminderter Wirkung von NO, wodurch das Auftreten von Komplikationen des Herz-Kreislaufsystems gefördert wird. Bei der Präeklampsie kommt es durch die Störung des NO-Stoffwechsels zu einer Verengung von Arterien, die einen Bluthochdruck bei der Mutter auslöst und durch Minderdurchblutung der Plazenta zur Gefährdung des ungeborenen Kindes führt. Ferner ist ein Mangel der Wirkungen von NO eine wesentliche Ursache der erektilen Dysfunktion. Dagegen ist SDMA zwar ebenfalls ein endogen vorkommendes Molekül, welches aber offenbar kleine inhibitorische Wirkung auf die Aktivität der NO-Synthase ausübt.

WO98/49199 offenbart ein Verfahren zum Nachweis einer Wahrscheinlichkeit des zukünftigen Auftretens oder Fortschreitens von koronarer Herzerkrankung, die mit einer Störung des NO-Stoffwechsels einhergeht, dadurch gekennzeichnet, dass ein Gehalt von endogenen Methyl-Argininen in einer biologischen Flüssigkeit nachgewiesen wird (Seite 4, Zeile 29 bis Seite 5, Zeile 17, Seite 6, Zeile 11 bis Zeile 22, Ansprüche 6 und 14).

Die vorliegende Erfindung wird durch den unabhängigen Anspruch 1 definiert. Bevorzugte Ausführungsformen sind in den Ansprüchen 2-21 zu finden.

Der Erfindung liegt die Beobachtung zugrunde, daß Patienten mit Hypercholesterinämie, peripherer Arteriosklerose, Herzinsuffizienz, chronischer Niereninsuffizienz, Diabetes mellitus und Hypertonie höhere Konzentrationen von ADMA im Plasma aufweisen als gesunde Probanden. Wir haben nunmehr erstmals zeigen können, daß ADMA ein für das zukünftige Fortschreiten der Erkrankungen prognostisch relevanter Faktor ist: Patienten mit höheren ADMA-Konzentrationen haben eine signifikant höhere Wahrscheinlichkeit, eine bedrohliche Durchblutungsstörung zu erleiden oder daran zu versterben als Patienten mit niedrigeren ADMA-Konzentrationen (Beispiel 1). Patienten mit hohen ADMA-Konzentrationen haben auch insgesamt ein signifikant höheres Risiko zu versterben, unabhängig von der zugrundeliegenden Todesursache (Beispiel 2). Ferner findet sich bei Patienten mit höheren ADMA-Konzentrationen signifikant häufiger eine Verdickung der Arterienwand in der Halsschlagader (A. carotis) als bei Patienten mit niedrigen ADMA-Konzentrationen (Beispiel 3). Patienten mit chronischer Herzinsuffizienz, die erhöhte ADMA-Konzentrationen aufweisen, haben eine geringere maximale Sauerstoffaufnahme unter körperlicher Belastung - ein Hinweis auf eine ungünstige Prognose des zukünftigen Krankheitsverlaufs (Beispiel 4).

Da ADMA somit ein für den Krankheitsverlauf der genannten Erkrankungen relevanter Faktor ist, ist es nützlich, die Konzentration beim individuellen Patienten mittels eines universell verfügbaren und raschen diagnostischen Tests spezifisch messen und sie von der SDMA-Konzentration unterscheiden zu können.

Die derzeit verfügbaren Meßverfahren zur quantitativen Bestimmung von ADMA und SDMA in Plasma, Serum, Urin und anderen biologischen Flüssigkeiten (Gewebsextrakten, Zellkulturüberständen u.a.) basieren allesamt auf dem chemischen Nachweisverfahren der Hochdruck-Flüssigkeitschromatografie (HPLC). Verschiedene Modifikationen von HPLC-Methoden werden hierzu derzeit eingesetzt. Diese Methoden sind jedoch sehr zeit- und personalintensiv, teuer, und somit für die klinische Routinediagnostik nicht geeignet.

Aufgabe der vorliegenden Erfindung ist es, eine Wahrscheinlichkeit des zukünftigen Fortschreitens von Erkrankungen nachzuweisen, die mit einer Störung des NO-Stoffwechsels einhergehen.

Diese Aufgabe wird erfindungsgemäß dadurch gelöst, daß ein Gehalt von endogenen Methyl-Argininen in biologischen Flüssigkeiten bestimmt wird.

Trotz der sehr umfänglichen Bestimmung verschiedenster bisher bekannter kardiovaskulärer Risikofaktoren ist bei einem Anteil von Patienten trotz Fehlens solcher bisher bekannter Risikofaktoren ein Fortschreiten der genannten Erkrankungen nachzuweisen. Die Erfindung weist den Vorteil auf, daß zumindest ein Teil dieser Fälle durch das Vorliegen erhöhter Konzentrationen der endogenen Methyl-Arginine erklärt werden kann und somit eine verbesserte Prognose über den Krankheitsverlauf abgegeben werden kann.

Die bisher eingesetzten Nachweisverfahren für ADMA und SDMA basieren auf dem Prinzip der HPLC, was sie sehr aufwendig und teuer macht, so daß diese Verfahren nicht umfassend in klinischen Alltag eingesetzt werden können, sondern spezialisierten Forschungslabors vorbehalten bleiben. Eine weitere Aufgabe der Erfindung ist es daher, ein Verfahren vorzulegen, mit dessen Hilfe einfach, kostengünstig und universell klinisch nutzbar die endogenen Methyl-Arginine ADMA und SDMA quantitativ in biologischen Flüssigkeiten bestimmt werden können.

Diese Aufgabe wird erfindungsgemäß dadurch gelöst, daß zur Bestimmung des Gehaltes von endogenen Methyl-Argininen in einer biologischen Flüssigkeit auf diese mit Antikörpern eingewirkt wird.

Die Bestimmung der ADMA- und SDMA-Konzentrationen mittels immunochemischer Verfahren (d.h. unter Verwendung von monoklonalen oder polyklonalen Antikörpern) hat erfindungsgemäß wesentliche Vorteile:
1. Die Messung der ADMA- und SDMA-Konzentrationen mittels Verwendung antikörperbasierter Verfahren muß nicht in hochspezialisierten HPLC-Labors durchgeführt werden. Immunochemische Nachweisverfahren wie Radioimmunoassays, Enzymimmunoassays etc. sind in den meisten klinischchemischen Labors routinemäßig verfügbar.
2. Die Messung mit immunochemischen Nachweisverfahren dauert weniger lange als die Messung mittels HPLC. Für letzteres Verfahren ist eine aufwendige Probenextraktion aus der jeweiligen Matrix (Plasma, Serum, Urin o.a.) erforderlich, damit die Messung per HPLC erfolgen kann. Das rasche Vorliegen des Meßwertes ist aber für die Verwendung des ADMA- bzw. SDMA-Nachweises im klinischen Alltag unabweisbar notwendig, damit dieser Parameter als Krankheits-Marker breite Akzeptanz finden kann.
3. Immunochemische Nachweisverfahren können neben der klinischen Routine auch in experimentellen Anwendungen zum Einsatz kommen (z.B. Immunohistochemie, Immunozytochemie, Immunoblotting).
4. Entsprechend der vorliegenden Erfindung ist der diagnostische Nachweis von ADMA bzw. SDMA geeignet, um prospektiv über das Erkrankungs- oder Todes-Risiko eines Patienten Aufschluß zu erlangen ("Risikofaktor").

Die Erzeugung monoklonaler Antikörper erfolgt nach allgemein bekannten immunologischen Verfahren durch Fusion von immunkompetenten Zellen sensibilisierter Versuchstiere mit Myelomzellen und Selektion der die spezifischen Antikörper produzierenden Zellklone mittels Standardverfahren. Die Erzeugung polyklonaler Antikörper erfolgt mittels Gewinnung von Immunserum von immunisierten Versuchstieren nach allgemein bekannten Verfahren. Die Produktion monoklonaler Antikörper gegen ADMA und SDMA umfaßt die Kultivierung der Antikörper-produzierenden Zellklone, die Gewinnung des Antikörper-enthaltenden konditionierten Mediums, sowie die Abfüllung und den Vertrieb der Antikörperlösungen. Die Nutzung der Antikörper umfaßt ihren Einsatz zu diagnostischen und wissenschaftlichen Zwecken, als Antikörper-Suspension oder als Bestandteil eines diagnostischen Kits, in den Bereichen der klinischen Medizin, der experimentellen Medizin, der Veterinärmedizin, der Biologie und anderer Biowissenschaften.

Die nachstehenden Beispiele erläutern die Erfindung:

### Beispiel 1

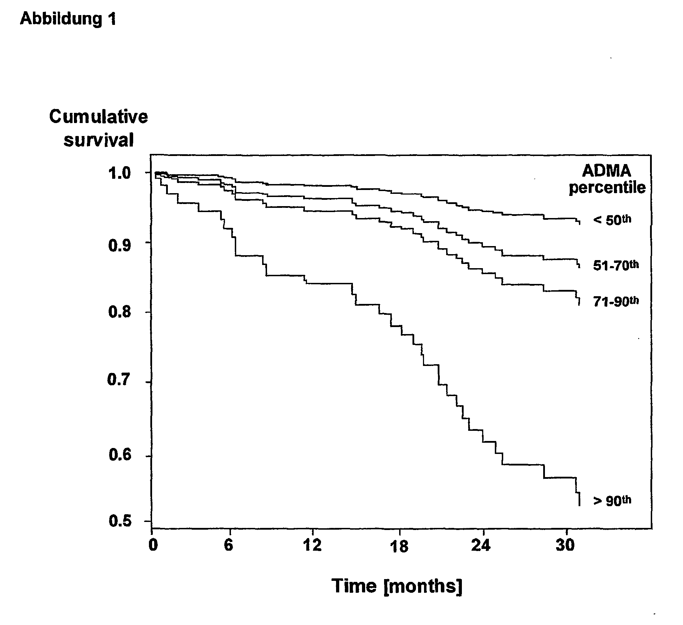

225 Patienten mit chronischer Niereninsuffizienz wurden in eine klinische Studie aufgenommen. Zu Beginn der Studie wurde jedem Patienten eine Blutprobe entnommen, in der die Konzentrationen von ADMA und SDMA sowie von L-Arginin bestimmt wurden. Die Patienten wurden über eine mittlere Beobachtungsdauer von 26 Monaten (1 - 35 Monaten) nachbeobachtet. Während dieser Zeit erlitten 57 Patienten ein kardiovaskuläres Krankheitsereignis (Myokardinfarkt, Schlaganfall, periphere Durchblutungsstörung oder kardial bedingter Tod). Patienten mit einem solchen kardiovaskulären Krankheitsereignis hatten zu Beginn der Studie signifikant höhere ADMA-Konzentrationen gehabt (Median 3,2 µmol/L) als Patienten ohne solches Ereignis (Median 2,2 µmol/L). ADMA erwies sich in der statistischen Analyse als ein unabhängiger Prädiktor kardiovaskulärer Krankheitsereignisse. Wurden die Patienten anhand der zu Beginn der Studie gemessenen ADMA-Plasmakonzuentrationen in Gruppen eingeteilt (ADMA < 50. Perzentile, 51. - 70. Perzentile, 71. - 90. Perzentile, und >90. Perzentile), so ergab sich eine klare Zunahme der Inzidenz kardiovaskulärer Todesfälle mit steigender ADMA-Konzentration (Abbildung 1).

### Beispiel 2

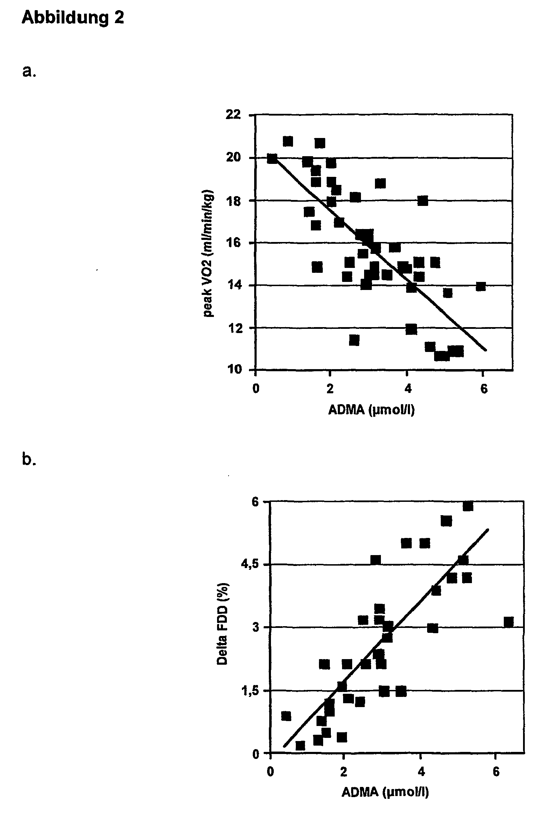

Bei 225 Patienten, bei denen in einer zu Beginn der Studie abgenommenen Blutprobe die ADMA-Konzentration bestimmt worden war, traten im Verlauf einer im Mittel 26 Monate dauernden Nachbeobachtungsphase insgesamt 57 Todesfälle aufgrund verschiedenster Ursachen auf. Patienten, die im Verlauf der Studie verstarben, hatten zu Beginn signifikant höhere ADMA-Konzentrationen aufgewiesen (Median 3,5 µmol/L) als Patienten, die überlebten (2,3 µmol/L). ADMA erwies sich als ein von anderen Risikofaktoren unabhängiger Prädiktor des Überlebens. Wurden die Patienten anhand der zu Beginn der Studie gemessenen ADMA-Plasmakonzentrationen in Gruppen eingeteilt (ADMA < 50. Perzentile, 51. - 70. Perzentile, 71. - 90. Perzentile, und >90. Perzentile), so ergab sich eine klare Zunahme der Gesamtmortalität mit steigender ADMA-Konzentration (Abbildung 2).

### Beispiel 3

Bei 90 Patienten wurde in einer Plasmaprobe die ADMA-Konzentration bestimmt. Anschließend wurde bei diesen Patienten mittels hochauflösender Ultraschalluntersuchung die Wanddicke der Halsschlagader (A. carotis) vermessen. ADMA war ein unabhängiger Einflußfaktor für die Intima/Media-Dicke der A. carotis (Abbildung 3), für die luminale Querschnittsfläche, und für den Schweregrad der arteriosklerotischen Läsionen in der A. carotis.

### Beispiel 4

Bei 45 Patienten mit chronischer Herzinsuffizienz wurde eine Blutprobe entnommen. Die Messung der ADMA-Konzentration ergab einen mittleren Wert von 4,1 ± 0,8 µmol/l, im Vergleich zu 1,0 ± 0,1 µmol/l bei gesunden Kontrollpersonen. Patienten mit höheren ADMA-Konzentrationen hatten eine geringere NO-vermittelte Gefäßdilatation (R = -0.79) und eine geringere maximale Sauerstoffaufnahme unter körperlicher Belastung (R = -0.81; Abbildung 2a). Die Gabe von L-Arginin führte zu einer signifikanten Verbesserung der NO-vermittelten Gefäßdilatation; das Ausmaß dieser Verbesserung durch L-Arginin war umso größer, je höher die ADMA-Konzentration beim jeweiligen Patienten war (R = 0.74; Abbildung 2b).

## Patentansprüche

1. Verfahren zur Prognose des kumulativen Überlebens von Patienten, **dadurch gekennzeichnet, dass** in einer Blutprobe eines erkrankten Patienten mit erhöhtem ADMA-Spiegel eine Konzentration von ADMA gemessen wird, um anhand einer empirisch ermittelten Eichkurve den Krankheitsverlauf des Patienten innerhalb eines Zeitraums von 0-30 Monaten zu bestimmen.

2. Verfahren nach Anspruch 1, **dadurch gekennzeichnet, dass** die Eichkurve mit steigender Konzentration der Methyl-Arginine eine sinkende Überlebenswahrscheinlichkeit des Patienten anzeigt.

3. Verfahren nach Anspruch 1 oder 2, **dadurch gekennzeichnet, dass** die Konzentration von endogenen Methyl-Argininen immunochemisch durch an diese spezifisch bindende Antikörper, vorzugsweise monoklonale Antikörper, bestimmt wird.

4. Verfahren nach einem der vorhergehenden Ansprüche, **dadurch gekennzeichnet, dass** die Antikörper als Lösung oder Suspension für diagnostische Zwecke verwendet werden.

5. Verfahren nach einem der vorhergehenden Ansprüche, **dadurch gekennzeichnet, dass** die Antikörper als Bestandteil eines diagnostischen Kits genutzt werden.

6. Verfahren nach einem der vorhergehenden Ansprüche, **dadurch gekennzeichnet, dass** die Erkrankung eine koronare Herzkrankheit ist.

7. Verfahren nach einem der vorhergehenden Ansprüche, **dadurch gekennzeichnet, dass** die Erkrankung eine chronische Herzinsuffizienz ist.

8. Verfahren nach einem der vorhergehenden Ansprüche, **dadurch gekennzeichnet, dass** die Erkrankung eine periphere arterielle Durchblutungsstörung ist.

9. Verfahren nach einem der vorhergehenden Ansprüche, **dadurch gekennzeichnet, dass** die Erkrankung eine chronische Niereninsuffizienz ist.

10. Verfahren nach einem der vorhergehenden Ansprüche, **dadurch gekennzeichnet, dass** die Erkrankung eine zerebrale ischämische Erkrankung ist.

11. Verfahren nach einem der vorhergehenden Ansprüche, **dadurch gekennzeichnet, dass** festgestellt wird, wie hoch in der biologischen Flüssigkeit eine Konzentration von ADMA ist.

12. Verfahren nach einem der vorhergehenden Ansprüche, **dadurch gekennzeichnet, dass** festgestellt wird, wie hoch in der biologischen Flüssigkeit ein Verhältnis der Konzentrationen von L-Arginin zu ADMA ist.

13. Verfahren nach einem der vorhergehenden Ansprüche, **dadurch gekennzeichnet, dass** festgestellt wird, wie hoch in der biologischen Flüssigkeit eine Konzentration von SDMA ist.

14. Verfahren nach einem der vorhergehenden Ansprüche, **dadurch gekennzeichnet, dass** festgestellt wird, wie hoch in der biologischen Flüssigkeit ein Verhältnis der Konzentrationen von L-Arginin zu SDMA ist.

15. Verfahren nach einem der vorhergehenden Ansprüche, **dadurch gekennzeichnet, dass** festgestellt wird, wie hoch in der biologischen Flüssigkeit ein Verhältnis der Konzentrationen von ADMA zu SDMA ist.

16. Verfahren nach einem der vorhergehenden Ansprüche, **dadurch gekennzeichnet, dass** die biologische Flüssigkeit Plasma ist.

17. Verfahren nach einem der vorhergehenden Ansprüche, **dadurch gekennzeichnet, dass** die biologische Flüssigkeit Serum ist.

18. Verfahren nach einem der vorhergehenden Ansprüche, **dadurch gekennzeichnet, dass** die biologische Flüssigkeit Urin ist.

19. Verfahren nach einem der vorhergehenden Ansprüche, **dadurch gekennzeichnet, dass** die biologische Flüssigkeit aus Gewebsextraktion besteht.

20. Verfahren nach einem der vorhergehenden Ansprüche, **dadurch gekennzeichnet, dass** die biologische Flüssigkeit aus histologischen Präparaten besteht.

21. Verfahren nach einem der vorhergehenden Ansprüche, **dadurch gekennzeichnet, dass** die biologische Flüssigkeit aus zytologischen Präparaten besteht.

## Claims

1. A method for the prognosis of the cumulative survival of patients, **characterized in that** an ADMA concentration is measured in a blood sample from a sick patient having an elevated ADMA level in order to be able to determine the course of the patient's disease over a period of 0-30 months on the basis of an empirical calibration curve.

2. The method according to Claim 1, **characterized in that** with an increasing methylarginine concentration, the calibration curve indicates a declining probability of survival of the patient.

3. The method according to Claim 1 or 2, **characterized in that** the concentration of endogenous methylarginines is determined immunochemically based on antibodies, preferably monocionale antibodies that bind specifically to methylarginines.

4. The method according to any one of the preceding claims, **characterized in that** the antibodies in the form of a solution or suspension are used for diagnostic purposes.

5. The method according to any one of the preceding claims, **characterized in that** the antibodies are used as a component of a diagnostic kit.

6. The method according to any one of the preceding claims, **characterized in that** the disease is coronary heart disease.

7. The methods according to any one of the preceding claims, **characterized in that** the disease is chronic heart failure.

8. The method according to any one of the preceding claims, **characterized in that** the disease is a peripheral arterial circulation disorder.

9. The method according to any one of the preceding claims, **characterized in that** the disease is chronic renal failure.

10. The method according to any one of the preceding claims, **characterized in that** the disease is a cerebral ischemic disease.

11. The method according to any one of the preceding claims, **characterized in that** it determines how high the concentration of ADMA in the biological fluid is.

12. The method according to any one of the preceding claims, **characterized in that** it determines how high the ratio of the concentrations of L-arginine to ADMA in the biological fluid is.

13. The method according to any one of the preceding claims, **characterized in that** it determines how high the concentration of SDMA in the biological fluid is.

14. The method according to any one of the preceding claims, **characterized in that** it determines how high the ratio of the concentrations of L-arginine to SDMA in the biological fluid is.

15. The method according to any one of the preceding claims, **characterized in that** it determines how high the ratio of the concentrations of ADMA to SDMA in the biological fluid is.

16. The method according to any one of the preceding claims, **characterized in that** the biological fluid is plasma.

17. The method according to any one of the preceding claims, **characterized in that** the biological fluid is serum.

18. The method according to any one of the preceding claims, **characterized in that** the biological fluid is urine.

19. The method according to any one of the preceding claims, **characterized in that** the biological fluid comprises tissue extracts.

20. The method according to any one of the preceding claims, **characterized in that** the biological fluid comprises histological preparations.

21. The method according to any one of the preceding claims, **characterized in that** the biological fluid comprises cytological preparations.

## Revendications

1. Procédé de pronostic de la survie cumulative de patients, **caractérisé en ce qu'**on mesure dans l'échantillon sanguin d'un patient malade à niveau ADMA accru une concentration d'ADMA afin de pouvoir déterminer à partir d'une courbe d'étalonnage déterminée empiriquement l'évolution pathologique du patient sur une durée de 0 à 30 mois.

2. Procédé selon la revendication 1, **caractérisé en ce que** la courbe d'étalonnage, en cas de concentration croissante de méthyle-arginine, indique une baisse de la probabilité de survie du patient.

3. Procédé selon la revendication 1 ou 2, **caractérisé en ce que** la concentration en méthyle-arginine endogène est définie par voie immunochimique par des anticorps se liant spécifiquement à celle-ci, de préférence des anticorps monoclonaux.

4. Procédé selon une des revendications précédentes, **caractérisé en ce que** les anticorps sont employés sous forme de solution ou suspension à des fins de diagnostic.

5. Procédé selon une des revendications précédentes, **caractérisé en ce que** les anticorps sont utilisés comme composante d'un kit de diagnostic.

6. Procédé selon une des revendications précédentes, **caractérisé en ce que** la pathologie est une maladie cardiaque coronaire.

7. Procédé selon une des revendications précédentes, **caractérisé en ce que** la pathologie est une insuffisance cardiaque chronique.

8. Procédé selon une des revendications précédentes, **caractérisé en ce que** la pathologie est une perturbation de la circulation sanguine artérielle périphérique.

9. Procédé selon une des revendications précédentes, **caractérisé en ce que** la pathologie est une insuffisance rénale chronique.

10. Procédé selon une des revendications précédentes, **caractérisé en ce que** la pathologie est une pathologie ischémique cérébrale.

11. Procédé selon une des revendications précédentes, **caractérisé en ce qu'**il est constaté quel est le niveau d'une concentration en ADMA dans le liquide biologique.

12. Procédé selon une des revendications précédentes, **caractérisé en ce qu'**il est constaté quel est le niveau du rapport entre les concentrations en L-arginine et en ADMA dans le liquide biologique.

13. Procédé selon une des revendications précédentes, **caractérisé en ce qu'**il est constaté quel est le niveau d'une concentration en SDMA dans le liquide biologique.

14. Procédé selon une des revendications précédentes, **caractérisé en ce qu'**il est constaté quel est le niveau du rapport entre les concentrations en L-arginine et en SDMA dans le liquide biologique.

15. Procédé selon une des revendications précédentes, **caractérisé en ce qu'**il est constaté quel est le niveau du rapport entre les concentrations en ADMA et en SDMA dans le liquide biologique.

16. Procédé selon une des revendications précédentes, **caractérisé en ce que** le liquide biologique est du plasma.

17. Procédé selon une des revendications précédentes, **caractérisé en ce que** le liquide biologique est du sérum.

18. Procédé selon une des revendications précédentes, **caractérisé en ce que** le liquide biologique est de l'urine.

19. Procédé selon une des revendications précédentes, **caractérisé en ce que** le liquide biologique est composé d'un extrait de tissu.

20. Procédé selon une des revendications précédentes, **caractérisé en ce que** le liquide biologique est composé de préparations histologiques.

21. Procédé selon une des revendications précédentes, **caractérisé en ce que** le liquide biologique est composé de préparations cytologiques.
